# EUROPÄISCHE PATENTSCHRIFT

(11) **EP 3 004 322 B1**
(45) Veröffentlichungstag und Bekanntmachung des Hinweises auf die Patenterteilung: **24.02.2021**
(21) Anmeldenummer: 14731164.1
(22) Anmeldetag: 23.05.2014
(51) Int. Cl.: C12M 1/107, C12M 1/00, C12M 1/12, C12M 1/34

(54) **VERFAHREN UND VORRICHTUNG FÜR DIE METHANISIERUNG VON GASEN MITTELS RIESELBETTREAKTOREN**
METHOD AND DEVICE FOR GAS METHANATION USING TRICKLE BED BIOREACTORS
PROCÉDÉ ET APPAREIL DE MÉTHANISATION DE GAZ AU MOYEN DE RÉACTEURS À LIT DE RUISSELLEMENT BACTÉRIEN

(30) Priorität: 24.05.2013 DE 102013209734
(43) Veröffentlichungstag der Anmeldung: 13.04.2016
(73) Patentinhaber: Brandenburgische Technische Universität Cottbus - Senftenberg, 03013 Cottbus (DE); Gicon Grossmann Ingenieur Consult GmbH, 01219 Dresden (DE)
(72) Erfinder: BUSCH, Günter, 01156 Dresden (DE); BURKHARDT, Marko, 03046 Cottbus (DE); GROSSMANN, Jochen, 01187 Dresden (DE)
(74) Vertreter: Dr. Klemens Schubert Patentanwalt
(86) Internationale Anmeldenummer: PCT/EP2014/060721
(87) Internationale Veröffentlichungsnummer: WO 2014/187985

(56) Entgegenhaltungen:
- WO-A1-2012/110256
- DE-A1- 2 103 647
- DE-A1- 3 842 295
- DE-A1-102008 037 402
- DE-A1-102010 043 630
- DE-A1-102011 051 836
- US-A- 4 921 799

## Beschreibung

Die vorliegende Erfindung betrifft ein Verfahren für die Methanisierung von Gasen mittels Rieselbettreaktoren sowie eine Vorrichtung zur Durchführung des Verfahrens.

Die Verwendung gasförmiger Substrate für die biochemische Herstellung von Methan ist, von einigen Laboranwendungen abgesehen, bisher noch nicht technisch eingeführt, gewinnt aber aufgrund neuerer energieressourcenwirtschaftlicher Strategien für die Nutzung von Kohlenstoffdioxid und elektrochemisch gewonnenem Wasserstoff an Bedeutung ("Power to Gas").

Gasförmige Substrate haben auch deshalb bisher keine Rolle für die Biogaserzeugung gespielt, weil ihr Mengenaufkommen als sehr gering eingeschätzt wurde und das Einpressen in Biogasanlagen auf technologische Probleme stößt: Es muss ein hoher Druck aufgebracht werden, um die Gase in die bisher üblichen, mit Gärflüssigkeit gefüllten Biogasreaktoren einzupressen, und es ist eine sehr gute Verteilung der Gasblasen erforderlich, um einen guten Stoffaustausch zwischen Gas und Flüssigkeit zu gewährleisten. Darüber hinaus ist die Verweilzeit der Gase nicht steuerbar.

Es sind bisher Anwendungen bekannt, bei denen zusätzlich zum üblichen Substrat (also nachwachsende Rohstoffe, Gülle u. ä.) ein Einpressen von Pyrolysegasen (JP 200-152 799), Wasserstoff (JP 06-169 783,
FR 2 537 992 A1, US 3 383 309 A, DE 10 2010 043 630 A1) oder Hydrolysegas (DE 10 2010 043 630 A1, US 4 696 746 A), in die Biogasreaktoren vorgenommen wird, um die Ausbeute an Methan zu steigern bzw. unerwünschte Gaskomponenten biologisch umzuwandeln.

In der US 4 351 729 A wird das Rieselbettprinzip für die anaerobe Behandlung eines Abwassers, dessen organische Inhaltsstoffe gleichzeitig zu vorzugsweise Methan abgebaut werden, eingesetzt. Eine Zuführung von Wasserstoff erfolgt allerdings nicht.

Des Weiteren ist aus der US 4 921 799 A ein Verfahren bekannt, in dem kohlenwasserstoffhaltige Gase, darunter auch ein CO₂-H₂-Gemisch, in Methan mittels eines Rieselbettreaktors umgewandelt werden können. Der Rieselbettreaktor besteht aus einem meist zylindrischen Reaktor, der mit einem Trägermedium (Schüttschicht, Packung), auf deren Oberfläche Methan bildende Mikroorganismen immobilisiert sind, und der vom umzuwandelnden Gas durchströmt wird. Die immobilisierten Mikroorganismen werden mittels eines herabrieselnden Flüssigkeitsfilms bzw. einer Nährstofflösung feucht gehalten und mit Nährstoffen versorgt. Am Ausgang dieses Reaktors wird das Produktgas, welches überwiegend aus Methan bestehen soll, abgezogen. Die Methanbildung kann auch unter einem erhöhten Systemdruck erfolgen.

Die oben genannten Probleme wurden bisher offenbar nicht gelöst. Das bisher bekannte und oben im Patent US 4 921 799 beschriebene Verfahren hat daher eine begrenzte Raum-Zeit-Ausbeute und eine unterhalb des Möglichen liegende Methankonzentration. Der Wasserstoffumsatz beträgt weniger als 60%, die Methanausbeute liegt bei maximal 56,5%. Beides sind jedoch entscheidende Kriterien für den wirtschaftlichen Einsatz der Methanisierung von Gasen, insbesondere bei der biochemischen Herstellung von Methan aus Kohlenstoffdioxid und Wasserstoff. Auch werden Reaktoren verwendet, deren Volumen lediglich 75 mL beträgt. Technische Anwendungen des Verfahrens nach US 4 921 799 sind jedenfalls nicht bekannt geworden, sodass offenbar auch kein durch die Praxis induzierter Forschungs- und Entwicklungsbedarf bestand.

Außerdem wird in der DE 10 2011 051 836 A1 ein Verfahren zur Erzeugung von Biogas unter der Verwendung eines Rieselbettreaktors beschrieben. Die immobilisierten Mikroorganismen befinden sich in einem Biofilm, der auf der Oberfläche von Aufwuchskörpern des Bioreaktors angebracht ist. Der Flüssigkeitsfilm, der die Mikroorganismen berieselt, enthält als Substrate Feststoffe, Flüssigkeiten und/oder Gase. Die Gase werden bei deren Einbringung in den Bioreaktor, in keinem festgelegten Mischverhältnis zueinander eingestellt.

In der WO 2008 094 282 A1 und der DE 10 2008 037 402 A1 werden jeweils Verfahren zur Umwandlung von Kohlenstoffdioxid und Wasserstoff in Methan unter der Verwendung eines mit Medium gefüllten Bioreaktors beschrieben. Diese Bioreaktortypen beziehen sich auf ein unter Wasserspannung stehendes Festbett. In der WO 2008 094 282 A1 werden ausschließlich methanbildende Archae verwendet, die nicht immobilisiert in der Flüssigkeit vorliegen. In der DE 10 2008 037 402 A1 werden immobilisierte methanbildende Bakterien in Suspension verwendet. In beiden Bioreaktoren gemäß dieser Druckschriften finden daher die Stoffübertragungen und die Gasführungen einzig in der Flüssigkeit statt.

Des Weiteren wird auch in der WO 2012 110 256 A1 die Produktion von Methan aus Kohlenstoffdioxid und Wasserstoff in einem vollständig gefluteten Bioreaktor beschrieben, bei dem der Stoffübergang und die Gasführung gänzlich in der Flüssigkeit stattfindet. Die methanbildenden Bakterien befinden sich in Suspension und sind nicht immobilisiert. Das Einbringen der Gase erfolgt über die Einstellung volumetrischer Mischungsverhältnisse in der Gasphase. Die Einstellung der zu lösenden gasförmigen Substrate in Bezug auf die stöchiometrischen Verhältnisse der Gase in der Prozessflüssigkeit erfolgt nicht.

Schließlich wird in der DE 38 42 295 A1 ein Tropfkörper zur Aufbereitung von Wasser offenbart, welcher beidseitig eine Vielzahl paarweise axial entgegengesetzt gerichteter Tropfstäbe trägt, die jeweils eine gemeinsame durch den Tropfkörper verlaufende Bohrung besitzt. Durch die Kapillarwirkung in der so gebildeten Vielzahl von Röhren sammeln sich in diesen die Mikroorganismen an, die beim Reinigen der Tropfkörper nicht herausgespült werden.

Aufgabe der vorliegenden Erfindung ist es daher, die im Stand der Technik vorhandenen Nachteile zu überwinden. Aufgabe der Erfindung ist es, ein Verfahren zur Methanisierung zur Verfügung zu stellen, bei dem die Raum-Zeit-Ausbeute an Methan wesentlich verbessert wird und welches den wirtschaftlichen Einsatz der Methanisierung von Gasen ermöglicht, insbesondere bei der biochemischen Herstellung von Methan aus Kohlenstoffdioxid und Wasserstoff.

Die Aufgabe der Erfindung wird durch ein Verfahren gelöst, welches die Merkmale des Hauptanspruchs aufweist. Vorteilhafte Ausgestaltungen des erfindungsgemäßen Verfahrens sind in den abhängigen Unteransprüche gekennzeichnet.

Gegenstand der vorliegenden Erfindung ist somit ein Verfahren zur Methanisierung gasförmiger Substrate, wobei man die folgenden Schritte ausführt:
a) Bereitstellen eines mit Aufwuchskörpern befüllten Reaktors mit auf der Oberfläche der Aufwuchskörper immobilisierten methanbildenden Mikroorganismen;
b) Berieseln der Aufwuchskörper mit den darauf immobilisierten methanbildenden Mikroorganismen mit einer Prozessflüssigkeit,
c) Beaufschlagen des Reaktors mit einer Mischung gasförmiger Substrate, wobei man das Mischungsverhältnis der Substrate in der Gasphase derart wählt, dass das Lösen der gasförmigen Substrate in der Prozessflüssigkeit zu einem stöchiometrischen Verhältnis der in der Prozessflüssigkeit gelösten gasförmigen Substrate führt,
d) Ableiten des erzeugten Gasgemisches aus dem Reaktor.

Besonders bevorzugt ist es dabei, dass man die gasförmigen Substrate auswählt aus Wasserstoff, Kohlenstoffdioxid, Kohlenwasserstoffen, Kohlenwasserstoffverbindungen, welche optionale Heteroatome, ausgewählt aus Sauerstoff, Stickstoff, Schwefel oder Halogenen, enthalten, und aus deren Mischungen.

Erfindungsgemäß bevorzugt ist es auch, dass man im Schritt a) zwei oder mehrere Reaktoren bereitstellt, wobei man die Reaktoren in Bezug auf den Gastransport in Reihe schaltet.

Bevorzugt ist das erfindungsgemäße Verfahren ferner, wobei man im Schritt b) die Prozessflüssigkeit in Bezug zur Transportrichtung der Gasmischung im Gegenstrom, im Gleichstrom, im Kreuzstrom oder in anderen Stromvarianten führt.

Besonders bevorzugt ist aber ein Verfahren, bei dem man im Schritt c) das Beaufschlagen des Reaktors mit der Mischung gasförmiger Substrate mit einem Überdruck durchführt, wobei der Druck 1 bis 100 bar oder darüber, vorzugsweise 1 bis 10 bar und besonders bevorzugt 1 bis 3 bar beträgt.

Weiterhin ist erfindungsgemäß bevorzugt, dass man im Schritt c) die Substratkonzentration in der Prozessflüssigkeit und/oder die Konzentration einzelner Bestandteile des erzeugten Gasgemisches am Ausgang des Reaktors misst, und dann das Mischungsverhältnis der gasförmigen Substrate wählt und einstellt.

Besonders bevorzugt ist ein Verfahren zur Methanisierung gasförmiger Substrate, bei dem man die Prozessflüssigkeit im Umlauf führt.

Weiterhin bevorzugt ist ein Verfahren zur Methanisierung gasförmiger Substrate, bei dem man die Prozessflüssigkeit auswählt aus Nährlösungen für methanbildende Mikroorganismen, extern erzeugten Hydrolysaten, Abläufen aus Biogasanlagen und aus deren Mischungen sowie aus Prozessflüssigkeiten, welche die erforderlichen Spurenelementkonzentrationen und/oder Nährstoffe aufweisen.

Die Aufgabe der Erfindung wird ferner durch das Bereitstellen einer Vorrichtung zur Durchführung des Verfahrens gelöst.

Gegenstand der vorliegenden Erfindung ist somit auch eine Vorrichtung mit den Merkmalen der nebengeordneten Vorrichtungsansprüche.

Gegenstand der Erfindung ist somit eine Vorrichtung, zur Durchführung des erfindungsgemäßen Verfahrens, umfassend mindestens zwei mit Aufwuchskörpern befüllte Reaktoren mit auf der Oberfläche der Aufwuchskörper immobilisierten methanbildenden Mikroorganismen, je Reaktor mindestens eine Berieselungsvorrichtung zum Berieseln der Aufwuchskörper mit einer Prozessflüssigkeit, wobei am Eingang und/oder am Ausgang der Berieselungsvorrichtung Messeinrichtungen zur Bestimmung mindestens eines Bestandteils der in der Prozessflüssigkeit gelösten gasförmigen Substrate vorgesehen ist,
je Reaktor mindestens eine Einleitvorrichtung zum Beaufschlagen des Reaktors mit einer Mischung gasförmiger Substrate, wobei diese optional mindestens eine Zufuhr für ein gasförmigen Substratbestandteil und/oder eine Mischeinrichtung für Gase aufweist, und
je Reaktor mindestens eine Ableitvorrichtung zum Ableiten des erzeugten Gasgemisches, wobei die Ableitvorrichtung optional in die Einleitvorrichtung eines benachbarten Reaktors mündet.

Gegenstand der Erfindung ist ferner auch eine Vorrichtung, zur Durchführung des erfindungsgemäßen Verfahrens, umfassend mindestens einen Reaktor mit mindestens einem im Wesentlichen zylinderförmigen Bereich, wobei der Reaktor in Bezug auf die Längsachse des zylindrischen Bereichs waagerecht angeordnet ist,
mindestens zwei Aufwuchskörper, welche als rotierbare Scheiben, die koaxial zueinander ausgebildet und in Richtung der Längsachse des zylindrischen Bereichs des Reaktors angeordnet sind,
eine Einleitvorrichtung zum Beaufschlagen des Reaktors mit einer Mischung gasförmiger Substrate, wobei diese optional mindestens eine Zufuhr für ein gasförmigen Substratbestandteil und/oder eine Mischeinrichtung für Gase aufweist,
eine Ableitvorrichtung zum Ableiten des erzeugten Gasgemisches.

Die vorliegende Erfindung wird mit den beigefügten Zeichnungen näher erläutert. Es zeigt:
Fig. 1a: ein Fließdiagramm einer ersten Ausführungsform einer Reihenschaltung von Rieselbettreaktoren mit Führung der Rieselflüssigkeit im Gegenstrom;
Fig. 1b: ein Fließdiagramm einer zweiten Ausführungsform einer Reihenschaltung von Rieselbettreaktoren mit Führung der Rieselflüssigkeit im Gleichstrom;
Fig. 1c: ein Fließdiagramm einer dritten Ausführungsform einer Reihenschaltung von Rieselbettreaktoren mit einer gestuften Zuführung von Kohlenstoffdioxid in die Reaktoren;
Fig. 2: ein Fließdiagramm einer weiteren Ausführungsform mit einer Kreuzstromschaltung von Rieselbettreaktoren; und
Fig. 3: eine perspektivische Ansicht von Rotationstauchkörperreaktoren.

Erfindungsgemäß wird ein Bioreaktor vorgeschlagen, der mit Aufwuchskörpern (Trägermedium aus hydrophobem Kunststoff mit großer volumenspezifischer Oberfläche) gefüllt ist, auf denen ein aktiver Biofilm immobilisiert wurde. Der Biofilm enthält dabei sowohl die Methanbakterien als auch die notwendige Begleitflora.

Dieses Trägermedium mit den darauf immobilisierten Mikroorganismen wird nun lediglich berieselt (analog zu einem Tropfkörper in der Abwassertechnik). Die gesamte im Reaktor befindliche Flüssigkeit bildet nunmehr einen langsam über das Trägermedium abwärts fließenden Flüssigkeitsfilm. Über diesen Flüssigkeitsfilm wird der Biofilm mit Wasser und mit Nährstoffen ausreichend versorgt. Durch den Wegfall der gespannten Flüssigkeit entfällt der statische Druck der Flüssigkeitssäule (etwa 1 bar pro 10 m Höhe). Der Druck im Inneren des Reaktors ist daher nahezu konstant und wird nur durch den von außen aufgeprägten Systemdruck und nicht durch die Flüssigkeitssäule bestimmt.

Das Zwischenraumvolumen im Reaktor ist nun flüssigkeitsleer und mit Gas gefüllt. Das (strömende) Gas hat somit einen sehr niedrigen Druckverlust. Die Verweilzeit des Gases wird nur durch den Gasstrom und nicht durch die Blasenaufstiegsgeschwindigkeit bestimmt und kann daher in beliebigen Größenordnungen gesteuert werden.

Erfindungsgemäß werden mehrere in dieser Art aufgebaute Reaktoren verwendet, die mit Bezug auf den Gastransport in Reihe geschaltet werden. Vor bzw. nach jedem dieser Reaktoren befindet sich ein Mischelement, mit dessen Hilfe die radial und in gewissen Grenzen auch axial auftretenden Konzentrationsunterschiede im Gas, vor allem nach der Zufuhr neuen Gases als Reaktionspartner, ausgeglichen werden. In dem nachfolgenden Reaktor wird ein homogenes Gasgemisch eingeleitet. Mit dieser Anordnung wird das Verweilzeitverhalten dem eines "idealen Strömungsrohres" angenähert und es wird eine nahezu konstante Verweilzeit der Gase erreicht. Der Umsatz der Gase und die Methankonzentration im Produktgas werden dadurch erhöht.

Die Zuführung der beiden Gase Kohlenstoffdioxid CO₂ und Wasserstoff H₂ erfolgt erfindungsgemäß nicht im stöchiometrischen Verhältnis, da aufgrund der unterschiedlichen Löslichkeiten dieses Verhältnis in der flüssigen Phase nicht aufrecht erhalten werden kann, sondern dort der Anteil des gelösten Kohlenstoffdioxids viel größer ist als der des gelösten Wasserstoffs. Darüber hinaus bewirkt die Lösung einer hohen Menge an CO₂ durch die Bildung von Kohlensäure ein Absenken des pH-Wertes in der Flüssigkeit und daher einen negativen Einfluss auf die methanogenen Mikroorganismen, welches sich einer geringeren Gasbildungsrate oder gar im Prozessversagen auswirken kann.

Erfindungsgemäß erfolgt daher am Eingang des Reaktors die Zugabe einer überstöchiometrischen Menge an H₂ und einer unterstöchiometrischen Menge an CO₂ derart, dass die in der Prozessflüssigkeit (=Rieselflüssigkeit) gelösten Gase im stöchiometrischen Verhältnis stehen. Die Berechnung der zum stöchiometrischen Verhältnis in der Flüssigkeit gehörigen Gaskonzentrationen kann über die jeweiligen Sorptionsisothermen der beiden Gase Wasserstoff und Kohlenstoffdioxid erfolgen, wobei vereinfachend nur das Kohlenstoffdioxid berücksichtigt werden braucht. Da dann in der Gasphase zwangsläufig ein großer Vorrat an Wasserstoff, jedoch nur ein kleiner Vorrat an Kohlenstoffdioxid vorhanden ist und dieser letztgenannte rasch umgesetzt wird, wird der beschriebene Effekt der Hemmung durch die Kohlensäure weitestgehend vermieden. Das Kohlenstoffdioxid muss durch eine Regeleinrichtung in jeder der einzelnen Stufen bzw. entlang des Reaktionswegs Kohlenstoffdioxid gemäß seines Umsatzes zugeführt werden.

Der geringe Partialdruck bzw. die geringe Löslichkeit des Wasserstoffs in der Flüssigkeit kann auch dadurch kompensiert werden, dass das gesamte System unter erhöhten Systemdruck gestellt wird. Gemäß des HENRY-Gesetzes nimmt dann die Konzentration des Wasserstoffs in der Flüssigkeit zu, sodass eine größere Menge an Wasserstoff pro Volumeneinheit an Flüssigkeit für die biochemische Methanbildung zur Verfügung steht. In gewissen Grenzen ist der spezifische Umsatz proportional zum Partialdruck des Wasserstoffs in der Flüssigkeit. Das trifft auch auf das Kohlenstoffdioxid zu, jedoch ist die Erhöhung seines Partialdrucks in der Flüssigkeit durch die gute Wasserlöslichkeit technisch nicht problematisch (s. o.). Die Zuführung des CO₂ sollte in der gleichen Art und Weise, wie oben beschrieben, geregelt erfolgen. Durch diese vorstehend beschriebene Bauweise, der Reihenschaltung mehrerer Reaktoren, und durch die gestufte Zuführung von Kohlenstoffdioxid erhöhen sich die Produktivität und der Umsatz des Verfahrens. Die Methankonzentration im Produktgas ist höher und erreicht Werte von > 99%. Die Schwankungen in der Methankonzentration sind sehr gering. Durch die Vermeidung einer Übersäuerung, hervorgerufen durch gelöstes CO₂, erhöht sich die Prozessstabilität.

Als Flüssigkeit für die Berieselung kann auch Ablaufflüssigkeit aus einer bestehenden Biogasanlage benutzt werden. Somit entfällt die Herstellung einer speziellen Nährflüssigkeit, da erfahrungsgemäß in dieser Ablaufflüssigkeit alle erforderlichen Nährstoffe für die methanogenen Mikroorganismen vorhanden sind.

Darüber hinaus bietet sich die Verwendung des Rieselbettprinzips auch für die Methanbildung aus Hydrolysat an, welches in einer vorgeschalteten Hydrolyse aus einem beliebigen Substrat (nachwachsende Rohstoffe, Abfälle etc.) gewonnen werden kann. In diesem Fall wird im Rieselbett ein Biogas der üblichen Zusammensetzung entstehen. Führt man zusätzlich Wasserstoff zu, so kann in einem Reaktor gleichzeitig die Gewinnung von zusätzlichem Methan vorgenommen werden, wobei das Kohlendioxid des Biogases als Kohlenstoffquelle dient. Auch mit diesem Verfahren sind Methangehalte > 95% erreichbar, allerdings muss hier mit einem höheren Anteil von Schadgasen, wie Schwefelwasserstoff und Ammoniak, gerechnet werden, die hydrolysatbürtig sind und nutzungsabhängig entfernt werden sollten.

Die Reaktoren können beliebige Formen haben, da sie lediglich das Gewicht des Trägermediums, nicht aber eine große Wassermenge zu tragen haben. Das erlaubt eine geringere Wandstärke, eine preiswertere Gründung sowie eine Gestaltung mit dem Ziel der optimalen Strömungsführung und des optimalen Stofftransports an der immobilisierten Biozönose. Darüber hinaus kann das Gas im Verhältnis zur Flüssigkeit im Gleichstrom, im Gegenstrom, im Kreuzstrom oder in anderen Stromvarianten geführt werden. Auch die Verwendung des Prinzips der aus der Abwassertechnik bekannten Scheibenreaktoren an anaerober Fahrweise ist vorteilhaft, wobei die Befeuchtung der auf den Scheiben immobilisierten Biozönose durch das periodische Eintauchen der Scheiben in ein Flüssigkeitsbad erfolgt.

Da mit dem entstehenden Methan erhebliche Mengen Wasser als Dampf abtransportiert werden, ist der Ersatz dieses Wassers in der Rieselflüssigkeit erforderlich. Dazu kann neben Frischwasser auch das Kondensat nachfolgender Kondensationsstufen zur Entfeuchtung des Methans genutzt werden. Die Korrektur der Zusammensetzung der Rieselflüssigkeit sowie deren gelegentlicher Austausch wegen einer Verschlammung können nach längerer Betriebszeit erforderlich werden.

Das Verblocken des Reaktors aufgrund des Anwachsens des Biofilmes im Langzeitbetrieb wird durch Einstellung eines entsprechenden Rieselregimes verhindert. Periodisch erfolgt die Reduktion der Biofilmdicke durch Erhöhung der grenzschichtnahen Scherspannung durch eine erhöhte Fließgeschwindigkeit (Rieselstrom/Gasstrom). Zusätzlich kann ein mechanischer Abrieb bzw. eine Biomasseausschleusung mittels angerauter Feststoffkugeln mit geringem Durchmesser erfolgen. Während des periodischen Spülvorganges (ca. 1 mal pro Jahr) werden mit dem Ablauf die Feststoffkugeln ausgetragen.

Das erfindungsgemäße Verfahren und die erfindungsgemäße Vorrichtung weisen gegenüber dem Stand der Technik erhebliche Vorteile auf, da die folgenden Nachteile des Standes der Technik gelöst werden.
I. Aufgrund der Durchströmung einer Schüttschicht oder einer Packung bildet sich kein homogenes Strömungsprofil mit einer konstanten Verweilzeit des Gases aus, sondern es liegt eine Verweilzeitverteilung des Gases vor. Das hat zur Folge, dass das Input-Gasgemisch nicht vollständig umgesetzt wird. Demzufolge finden sich im Output- bzw. Produktgas immer noch Anteile des Input-Gasgemisches. Das ist gleichbedeutend mit einem unvollständigem Umsatz des Input-Gasgemisches und einer niedrigen Produktgaskonzentration.
II. Für die wirtschaftlich bedeutsame biochemische Methansynthese aus Wasserstoff und Kohlenstoffdioxid (4H₂ + CO₂ => CH₄ + 2H₂O) ergeben sich folgende, die Produktivität hemmende Schwierigkeiten: Der Wasserstoff hat eine sehr geringe Löslichkeit in Wasser. Daher wird der Umsatz zu Methan wesentlich durch den diffusiven Transport des Wasserstoffs aus dem Gas über den herabrieselnden Flüssigkeitsfilm in den Biofilm aus immobilisierten Methanbildnern bestimmt. Dieser Wasserstofftransport ist daher intensitätslimitierend für die Produktbildung. Kohlenstoffdioxid löst sich hervorragend im Wasser. Dessen Transport aus dem Gas in den Biofilm stellt keine Limitierung dar und die Flüssigkeit ist praktisch immer an Kohlenstoffdioxid gesättigt. Das Wasserstoff-Kohlendioxid-Gemisch wird im Patents US 4 921 799 beschrieben. Das beschriebene Verfahren speist jedoch in einem Molverhältnis der Gase (H₂:CO₂ >1 und nicht ausdrücklich im stöchiometrischen Molverhältnis 8:44) in den Reaktor ein. Durch die unterschiedlichen Sorptionsgleichgewichte von Kohlenstoffdioxid und Wasserstoff kommt es zwangsläufig zu einem unterstöchiometrischen Wasserstoffangebot an die Methanbildner, die somit ihre maximale Syntheseleistung für Methan nicht erreichen können. Diese wäre nur dann gegeben, wenn nicht in der Gasphase, sondern im Flüssigkeitsfilm das stöchiometrische Verhältnis von gelöstem Wasserstoff und gelöstem Kohlenstoffdioxid vorhanden sein würde.
III. Als Konsequenz der guten Löslichkeit von Kohlenstoffdioxid in der Flüssigkeit versäuert die Flüssigkeit durch die Bildung von Kohlensäure H₂CO₃. Der pH-Wert sinkt dabei deutlich unter den für die Methanisierung optimalen Bereich von 6,8-8. Daher sinkt auch die Biogasproduktion. Wegen der gegenüber einem konventionellem Fermenter sehr geringen Flüssigkeitsmenge in diesem Reaktor ist die Pufferkapazität des Kohlensäure-Hydrogenkarbonat-Kohlenstoffdioxid-Puffers rasch erschöpft und kann kaum Protonen zur Pufferung des pH-Wertes aufnehmen.
IV. Die Verweilzeit der Flüssigkeit, die an dem Trägermedium herabrieselt, kann nicht beeinflusst werden und ist nicht konstant, sondern ergibt sich aus dem örtlich und zeitlich veränderlichen Gleichgewicht zwischen Gravitationskraft und der Summe aller Strömungswiderstände. Es gibt daher Volumenbereiche innerhalb des Reaktors, die ungenügend mit Flüssigkeit benetzt werden und in denen die methanogenen Mikroorganismen unterversorgt sind, neben solchen Bereichen, in denen die Flüssigkeit in einer Kanalströmung bevorzugt herabrieselt und den Biofilm von dem Trägermedium abspült. Beide Fälle bewirken eine nachteilige Beeinträchtigung der Methanisierung.
V. Als Flüssigkeit zur Berieselung wird eine dafür hergestellte Nährstoffflüssigkeit verwendet. Die Inputgase werden außerhalb des Reaktors erzeugt oder bereitgestellt. Eine Methananreicherung eines im Prozess generierten Biogases, bei dem für die Berieselung keine Nährflüssigkeit, sondern ein Hydrolysat verwendet und aus dem gleichzeitig Biogas erzeugt wird, ist ebenfalls möglich. Eine zusätzliche Einspeisung von Wasserstoff ist bei diesen Verfahren nicht bekannt.
VI. Die Biofilmentwicklung auf dem Trägermedium führt im Langzeitbetrieb zu einer Verblockung des Reaktors bzw. zur Ausbildung einer mehr oder weniger ausgeprägten Kurzschlussströmung. Eine starke Verminderung der Raum-Zeit-Ausbeute resultiert daraus.

Durch das erfindungsgemäße Verfahren und die erfindungsgemäße Vorrichtung werden die unter I. bis VI. genannten Nachteile überwunden. Im folgenden werden das erfindungsgemäße Verfahren und die erfindungsgemäße Vorrichtung detailliert anhand verschiedener Ausführungsformen ausführlich beschrieben. Es ist klar, dass die in den Figuren beschriebenen Ausführungsbeispiele den Umfang der Erfindung lediglich erläutern, diesen aber nicht beschränken. Der Fachmann kann diese Ausführungsbeispiele ohne erfinderisches Zutun abwandeln, ohne dabei den erfinderischen Gedanken zu verlassen.

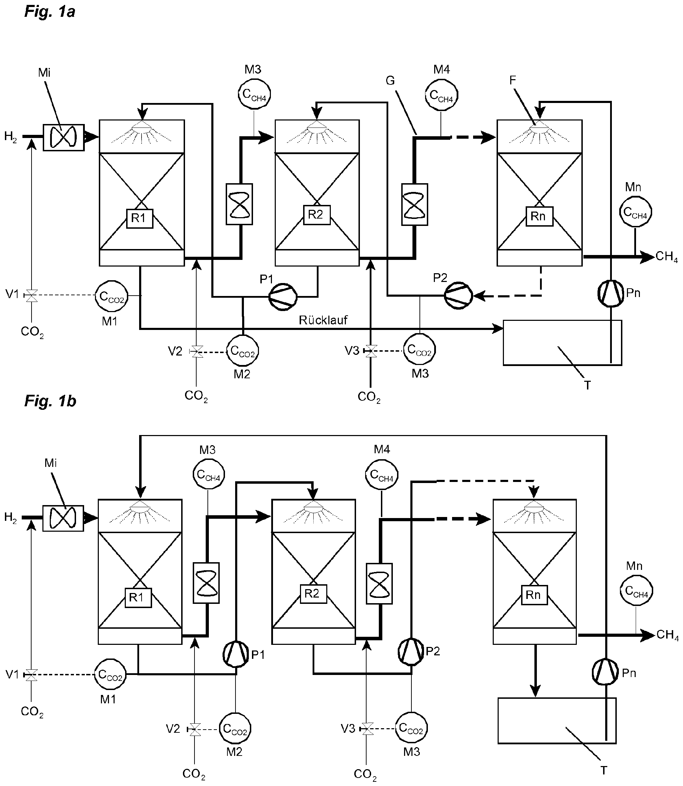

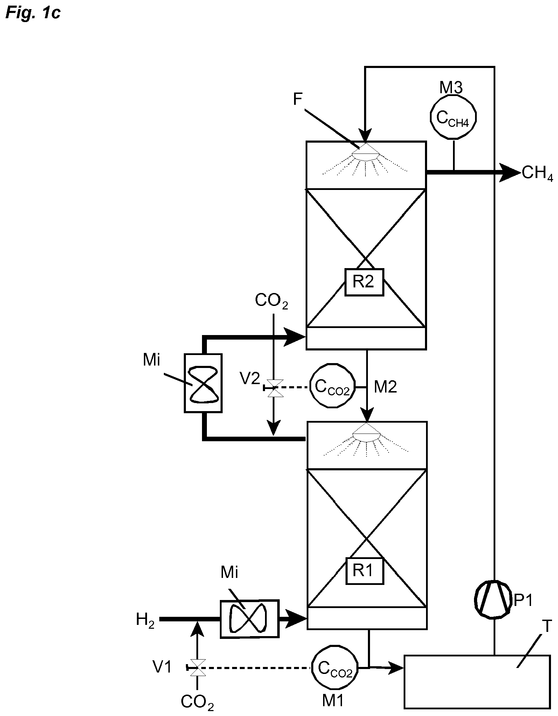

In den Figuren 1 bis 1c sind verschiedenen Ausführungsformen des erfindungsgemäßen Verfahrens in Form von Fließdiagrammen dargestellt.

Es werden mehrere als Druckbehälter ausgelegte, zylindrische Reaktoren R1 bis Rn mit einem Trägermedium befüllt, auf dem die vorher oder auch nach der Befüllung Methan bildenden Mikroorganismen immobilisiert wurden. Diese Reaktoren werden mit Gas führenden Rohrleitungen derart verbunden, dass ein Gas die Reaktoren nacheinander durchströmt. An oder in diesen Rohrleitungen befinden sich statische Mischer, die für den Ausgleich von Konzentrationsdifferenzen im Gas sorgen und es so homogenisieren. Es werden Gaszuführungen für die Zuführung von Kohlenstoffdioxid CO₂ über die Ventile V1 bis V3 an diese Rohrleitungen derart angebracht, dass das CO₂ dem in den Rohren strömenden Gas vor den statischen Mischern zugeführt werden kann. Eine derartige Zuführung soll auch am Eintritt des Gases, hier Wasserstoff H₂, in den ersten Reaktor vorhanden sein. Alle Reaktoren können unter erhöhtem Druck betrieben werden. Dadurch wird die Raum-Zeit-Ausbeute an entstehendem Methan erhöht, d.h. pro Einheit des Reaktorvolumens wird mehr Methan in kürzerer Zeit produziert.

Das in den zylindrischen Reaktoren befindliche Trägermedium wird durch eine im Kreislauf geführte Flüssigkeit berieselt, die entweder eine Nährflüssigkeit, ein Hydrolysat oder Methanablauf eines anderen Biogasreaktors oder Fermenters sein kann. Am Ablauf dieser Flüssigkeit aus den Reaktoren sind Messfühler M1 bis M3 angeordnet, die die Konzentration bzw. den Partialdruck des CO₂ in der Flüssigkeit messen können. Diese Messfühler regeln, ggf. über eine elektronische Regeleinheit, die jeweils zugeordneten Ventile V1 bis V3 derart, dass die Konzentration des CO₂ in der ablaufenden Rieselflüssigkeit einem (niedrigen) Voreinstellwert entspricht. Damit kann regelungstechnisch der optimale Arbeitspunkt der jeweiligen Reaktoren, gekennzeichnet durch den maximalen Umsatz an CO₂ mit H₂, eingestellt werden. Gleichzeitig wird die Versäuerung der Riesel- bzw. Nährflüssigkeit verhindert.

Mit der Messung der Gaskonzentration an den Messpunkten M3 und M4 kann die Zuführung der beiden Gase H₂ und CO₂ darüber hinaus an die üblicherweise steigende Leistungsfähigkeit der methanogenen Mikroorganismen dynamisch über einen Regelalgorithmus angepasst werden. So kann auch der Austritt nicht umgesetzter Reaktionspartner verhindert und die Methankonzentration im Produktstrom maximiert werden.

Der Transport der Rieselflüssigkeit von einem Rieselbettreaktor zum anderen kann in Bezug zur Transportrichtung des Gases im Gegenstrom (Fig. 1a), im Gleichstrom (Fig. 1b) oder im Kreuzstrom (Fig. 1c) mittels der Pumpen P1 bis Pn erfolgen. Die Rieselflüssigkeit wird am Kopf der Rieselbettreaktoren mittels einer Verteilvorrichtung gleichmäßig über die Rieselkörper verteilt. Dabei ist eine kontinuierliche Berieselung nicht zwingend erforderlich, sondern kann auch durch eine periodische Berieselung ersetzt werden.

Der Betrieb im Gegenstrom hat den Vorteil eines besseren Stoffübergangs zwischen Gas und Flüssigkeit aufgrund höherer Konzentrations- bzw. Partialdruckdifferenzen, sodass die Raum-Zeit- Ausbeute steigt. Beim Betrieb im Gleichstrom kann die Zuführung von Kohlenstoffdioxid vor dem in Bezug auf den Gasstrom letzten Rieselbettreaktor (hier: Rn) entfallen, sodass nicht nur verbleibendes CO₂ nahezu vollständig umgesetzt wird, sondern mögliche Restgehalte an CO₂ auch mit der Rieselflüssigkeit ausgewaschen werden. Die Gleichstromfahrweise hat daher den Vorteil einer höheren Produktqualität. Beide Fahrweisen können auch gekoppelt werden, z.B. derart, dass die in Gasrichtung ersten Rieselbettreaktoren im Gegenstrom, die letzten jedoch im Gleichstrom betrieben werden.

Die Steuerung der gestuften CO₂-Zugabe ist nicht erforderlich bzw. nicht sinnvoll, falls als Eintrittsgas Biogas oder/und als Rieselflüssigkeit ein Hydrolysat verwendet werden soll. In diesen Fällen ist das CO₂ bereits Bestandteil des eintretenden Gases bzw. wird im Füllköperreaktor durch mikrobiellen Abbau selbst erzeugt. In diesem Fall sollte Wasserstoff geregelt zugegeben werden, wobei die Zugabe von Wasserstoff so gesteuert wird, dass kein oder nur sehr geringe Mengen im Produktgas enthalten sind.

Am Gasaustritt der Reaktoren werden mittels Gassensoren die Gaszusammensetzung, insbesondere die Konzentrationen bzw. Partialdrücke von Wasserstoff H₂, Kohlenstoffdioxid CO₂ und ggf. weiteren Gasen, gemessen. Mit diesen Informationen können die Funktionsfähigkeit und die Leistung der einzelnen Reaktoren überwacht werden.

Es ist auch apparatetechnisch möglich, die Rieselbettreaktoren übereinander anstatt nebeneinander anzuordnen. Eine mögliche Variante dazu zeigt Fig. 1c. Diese Anordnung ist platzsparend und es sind weniger Pumpen erforderlich, falls die Gravitationskraft für die Verteilung der Flüssigkeit ausreicht. Hier kann ggf. auch auf die in der Fig. 1c dargestellten extern angeordneten statischen Mischer verzichtet und es kann auch das CO₂ unmittelbar, jedoch geregelt zwischen den Reaktoren zugeführt werden.

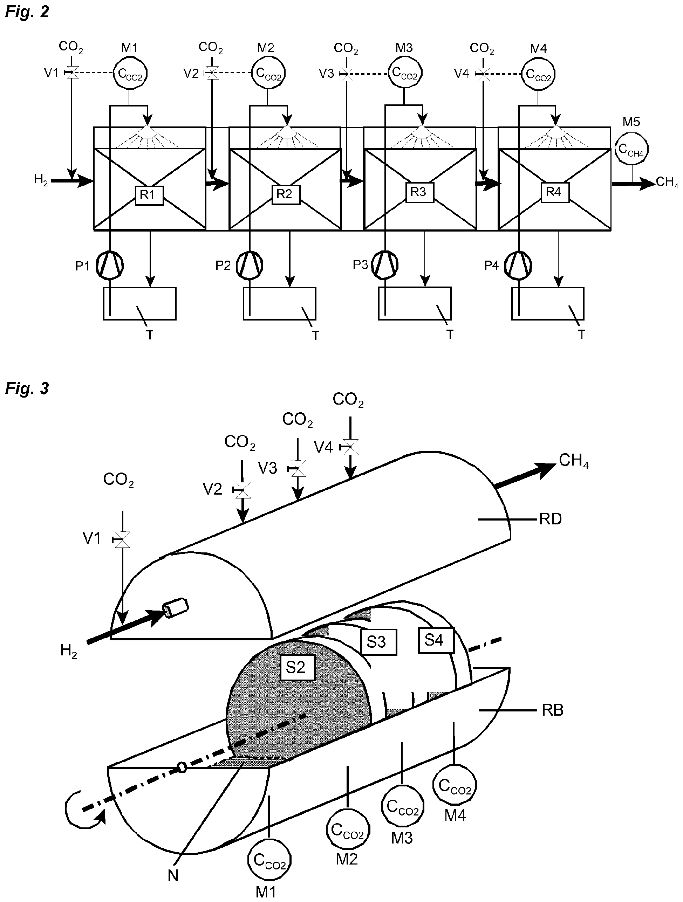

Fig. 2 zeigt den Betrieb der Rieselbettreaktoren im Kreuzstrom. Diese können baulich in einer druckfesten Einheit, z.B. in einem rechteckigen, liegenden Kanal, angeordnet werden. Das Gas strömt durch eine Reihenschaltung oder durch einen langgestreckten Reaktor in hauptsächlich waagerechter Richtung. Dabei können allerdings Umlenkvorrichtungen das Gas örtlich auch in andere Richtungen lenken, sodass der Gasweg verlängert und die axiale Rückvermischung vermindert werden. Die Rieselflüssigkeit wird oberhalb des Trägermediums zugegeben und rieselt an ihnen senkrecht herab. Die Hauptrichtungen des Gasstroms und der Rieselflüssigkeit stehen im rechten Winkel zueinander, sie kreuzen sich.

Fig. 3 zeigt die Verwendung, der an sich aus der Abwasserbehandlung bekannten Rotationstauchkörper für Methanisierung. Während sie allerdings dort in der Regel aerob betrieben werden, ist hier der anaerobe Betrieb zwingend erforderlich. Die Zuführung der Gase erfolgt in Analogie zu den oben beschriebenen Verfahrensweisen stufenweise. Der technologische Vorteil der Rotationstauchkörper besteht darin, dass die Berieselung durch die Benetzung der perforierten, gasdurchlässigen Tauchkörper, auf denen die Methanbildner immobilisiert sind, durch ständig wiederholtes Eintauchen in die Nährflüssigkeit bei der Rotation erfolgt. Das Gas strömt axial durch die perforierten Scheiben und wird dort biochemisch umgewandelt. Die Messstellen M1 bis M4 steuern die Ventile V1 bis V4 gemäß des oben beschriebenen Algorithmus.

Die gasförmigen Substrate werden in der Gasphase erfindungsgemäß derart eingestellt, dass das Lösen der gasförmigen Substrate in der Prozessflüssigkeit zu einem stöchiometrischen Verhältnis der in der Prozessflüssigkeit gelösten gasförmigen Substrate führt. Die Einstellung des stöchiometrischen Verhältnisses der gelösten Gase im Rieselfilm erfolgt unter Berücksichtigung der jeweiligen Gleichgewichtsbedingung. Zusätzlich kann durch einen erhöhten Systemdruck auf das gesamte System der geringere Partialdruck des Wasserstoffes kompensiert werden und die Konzentration des Wasserstoffes nimmt gemäß dem HENRY-Gesetz zu.

Daraus resultierend liegt weniger Kohlenstoffdioxid in der Prozessflüssigkeit vor. Daher tritt eine Hemmung der Kohlensäurebildung ein was zur Folge hat, dass keine Absenkung des pH-Wertes eintritt. Der negative Einfluss auf die methanbildenden Bakterien bleibt aus. Außerdem befindet sich mehr Wasserstoff in der Prozessflüssigkeit, wodurch die Methanbildung zusätzlich erhöht wird.

Die Umsatzreaktion der Gase findet im Wesentlichen an der Grenzfläche bzw. im Volumen des Biofilmes statt.

Die erfindungsgemäße Verwendung eines Rieselbettreaktors führt, wegen der Einstellung von Gleichgewichtsbedingungen zwischen Gas- und Flüssigphase, zur Lösung der eingebrachten Gase im Rieselfilm unter Ausbildung eines Konzentrationsgradienten. Aufgrund der Betriebsweise als Rieselbettreaktor mit dem Gasgemisch als Kontinuum, kann der Gasdurchsatz technisch exakt gesteuert werden. Daher werden der Gasdurchsatz und folglich auch die Verweilzeit des Gases nicht von der Blasenaufstiegsgeschwindigkeit bestimmt, wie dies bei Festbettreaktoren der Fall ist. Aufgrund der Blasenaufstiegsgeschwindigkeit kam es im Stand der Technik in beschriebenen Verfahren regelmäßig zu unvollständigen Umsätzen der Reaktionspartner. Um dies zu vermeiden, mussten die Reaktionspartner dann mehrfach als Gas in den Reaktor eingepresst werden. Dies führt jedoch zu einen erhöhtem Aufwand und zu einem geringeren Durchsatz. Demnach ist die Anwendung solcher Systeme begrenzt.

Des Weiteren bleibt der hydrostatische Druck der Flüssigkeitssäule (Höhe der Füllung im Fermenter) bei Rieselbettreaktoren aus. Das beruht darauf, dass in einem Rieselbettreaktor keine Flüssigkeitssäule mehr durch gespannte Flüssigkeit, wie in einem vollständig gefluteten Bioreaktor, vorliegt. Daher entfällt auch die Erzeugung eines zusätzlichen Gasdrucks zur Überwindung dieses hydrostatischen Drucks. In einem erfindungsgemäßen Rieselbettreaktor bildet die Flüssigkeit einen langsam über das Trägermedium fließenden Flüssigkeitsfilm aus.

Die mit dem erfindungsgemäßen Verfahren und der erfindungsgemäßen Vorrichtung erzielten Wasserstoffumsätze liegen zwischen 95 bis 100% und die Methanausbeute beträgt hierbei mehr als 99%. Die Werte liegen deutlich über denen, die im Stand der Technik bekannt sind.

Die Vorteile des beschriebenen erfindungsgemäßen Verfahrens und der beschriebenen erfindungsgemäßen Vorrichtung sind die Folgenden:
- Ersatz des einstufigen Festbettreaktors durch mehrere, in Bezug auf den Gasstrom in Reihe geschaltete, Rieselbettreaktoren mit dazwischen liegenden Mischelementen.
- Gestufte Einspeisung des für die Reaktion erforderlichen Kohlenstoffdioxids CO₂ in die Reaktoren derart, dass die in der Flüssigkeit gelösten Gase CO₂ und H₂ ständig im stöchiometrischen Verhältnis vorliegen, und dass eine Versäuerung durch gelöstes CO₂ vermieden wird.
- Verwendung von Ablauf einer Biogasanlage als Rieselflüssigkeit und somit Einsparung einer speziellen Nährlösung.
- Methanisierung eines extern erzeugten Hydrolysats zu Biogas und Zuführung von Wasserstoff in ein Rieselbett bzw. in die oben beschriebene Reihenschaltung mehrerer solcher Reaktoren zur Erhöhung der Methankonzentration.
- Nutzung rotierender Tauchkörper, z.B. Scheibenreaktoren, anstelle von berieselten Schüttschichten und dadurch Wegfall von Pumpen.

Die erfindungsgemäße Vorrichtung und das erfindungsgemäße Verfahren können in folgenden Anwendungsgebieten eingesetzt werden:
- Unternehmen, die das Prinzip "Power to Gas" umsetzen wollen.
- Hersteller von Biogasanlagen, die mit z.B. Füllkörpern in der Methanstufe ausgerüstet sind.
- Betreiber von Windkraftanlagen, die überschüssige Elektroenergie über eine Elektrolyse in Wasserstoff und diesen in Methan wandeln wollen.
- Kohlekraftwerksbetreiber, die CO₂ nicht ablagern, sondern zumindest teilweise verwerten wollen.
- Produzenten von organikreichen Abgasen.
- Betreiber von Biogasanlagen, die bestehende Anlagen um eine Methananreicherung aufrüsten wollen.

### Bezugszeichenliste

- R1, R2, R3, R4, Rn: Rieselbettreaktor
- M1, M2, M3, M4, M5: Messstelle
- P1, P2, P3, P4, Pn: Pumpe
- V1, V2, V3, V4: Ventil
- Mi: Mischer
- T: Tank
- F: Flüssigkeitsverteilung
- G: Gasstrom
- S2, S3, S4: Aufwuchskörper
- N: Prozessflüssigkeit
- RD: Reaktordeckel
- RB: Reaktorboden

## Patentansprüche

1. Verfahren zur Methanisierung gasförmiger Substrate, wobei man die folgenden Schritte ausführt:
a) Bereitstellen eines mit Aufwuchskörpern befüllten Reaktors mit auf der Oberfläche der Aufwuchskörper immobilisierten methanbildenden Mikroorganismen;
b) Berieseln der Aufwuchskörper mit den darauf immobilisierten methanbildenden Mikroorganismen mit einer Prozessflüssigkeit,
c) Beaufschlagen des Reaktors mit einer Mischung gasförmiger Substrate, wobei man das Mischungsverhältnis der Substrate in der Gasphase derart wählt, dass das Lösen der gasförmigen Substrate in der Prozessflüssigkeit zu einem stöchiometrischen Verhältnis der in der Prozessflüssigkeit gelösten gasförmigen Substrate führt,
d) Ableiten des erzeugten Gasgemisches aus dem Reaktor.

2. Verfahren zur Methanisierung gasförmiger Substrate, gemäß Anspruch 1, **dadurch gekennzeichnet, dass** man die gasförmigen Substrate auswählt aus Wasserstoff, Kohlenstoffdioxid, Kohlenwasserstoffen, Kohlenwasserstoffverbindungen, welche optionale Heteroatome, ausgewählt aus Sauerstoff, Stickstoff, Schwefel oder Halogenen, enthalten, und aus deren Mischungen.

3. Verfahren zur Methanisierung gasförmiger Substrate, gemäß Anspruch 1 oder 2, **dadurch gekennzeichnet, dass** man im Schritt a) zwei oder mehrere Reaktoren bereitstellt, wobei man die Reaktoren in Bezug auf den Gastransport in Reihe schaltet.

4. Verfahren zur Methanisierung gasförmiger Substrate, gemäß Anspruch 3, **dadurch gekennzeichnet, dass** man im Schritt b) die Prozessflüssigkeit in Bezug zur Transportrichtung der Gasmischung im Gegenstrom, im Gleichstrom, im Kreuzstrom oder in anderen Stromvarianten führt.

5. Verfahren zur Methanisierung gasförmiger Substrate, gemäß einem der Ansprüche 1 bis 4, **dadurch gekennzeichnet, dass** man im Schritt c) das Beaufschlagen des Reaktors mit der Mischung gasförmiger Substrate mit einem Überdruck durchführt, wobei der Druck 1 bis 100 bar, vorzugsweise 1 bis 10 bar und besonders bevorzugt 1 bis 3 bar beträgt.

6. Verfahren zur Methanisierung gasförmiger Substrate, gemäß einem der Ansprüche 1 bis 5, **dadurch gekennzeichnet, dass** man im Schritt c) die Substratkonzentration in der Prozessflüssigkeit und/oder die Konzentration einzelner Bestandteile des erzeugten Gasgemisches am Ausgang des Reaktors misst, und dann das Mischungsverhältnis der gasförmigen Substrate wählt und einstellt.

7. Verfahren zur Methanisierung gasförmiger Substrate, gemäß einem der Ansprüche 1 bis 6, **dadurch gekennzeichnet, dass** man die Prozessflüssigkeit im Umlauf führt.

8. Verfahren zur Methanisierung gasförmiger Substrate, gemäß einem der Ansprüche 1 bis 7, **dadurch gekennzeichnet, dass** man die Prozessflüssigkeit auswählt aus Nährlösungen für methanbildende Mikrorganismen, extern erzeugten Hydrolysaten, Abläufen aus Biogasanlagen und aus deren Mischungen sowie aus Prozessflüssigkeiten, welche die erforderlichen Spurenelementkonzentrationen und/oder Nährstoffe aufweisen.

9. Vorrichtung, zur Durchführung eines Verfahrens gemäß Anspruch 1, umfassend mindestens
zwei mit Aufwuchskörpern befüllte Reaktoren mit auf der Oberfläche der Aufwuchskörper immobilisierten methanbildenden Mikroorganismen,
je Reaktor mindestens eine Berieselungsvorrichtung zum Berieseln der Aufwuchskörper mit einer Prozessflüssigkeit, wobei am Eingang und/oder am Ausgang der Berieselungsvorrichtung Messeinrichtungen zur Bestimmung mindestens eines Bestandteils der in der Prozessflüssigkeit gelösten gasförmigen Substrate vorgesehen ist,
je Reaktor mindestens eine Einleitvorrichtung zum Beaufschlagen des Reaktors mit einer Mischung gasförmiger Substrate, wobei diese optional mindestens eine Zufuhr für ein gasförmigen Substratbestandteil und/oder eine Mischeinrichtung für Gase aufweist, und
je Reaktor mindestens eine Ableitvorrichtung zum Ableiten des erzeugten Gasgemisches, wobei die Ableitvorrichtung optional in die Einleitvorrichtung eines benachbarten Reaktors mündet.

10. Vorrichtung, zur Durchführung eines Verfahrens gemäß Anspruch 1, umfassend mindestens
einen Reaktor mit mindestens einem im Wesentlichen zylinderförmigen Bereich, wobei der Reaktor in Bezug auf die Längsachse des zylindrischen Bereichs waagerecht angeordnet ist,
mindestens zwei Aufwuchskörper, welche als rotierbare Scheiben, die koaxial zueinander ausgebildet und in Richtung der Längsachse des zylindrischen Bereichs des Reaktors angeordnet sind,
eine Einleitvorrichtung zum Beaufschlagen des Reaktors mit einer Mischung gasförmiger Substrate, wobei diese optional mindestens eine Zufuhr für ein gasförmigen Substratbestandteil und/oder eine Mischeinrichtung für Gase aufweist,
eine Ableitvorrichtung zum Ableiten des erzeugten Gasgemisches.

## Claims

1. Method for methanation of gaseous substrates, wherein the following steps are carried out:
a) providing a reactor filled with growth bodies carrying immobilized methane-forming microorganisms on the surface of the growth bodies;
b) trickling the growth bodies with the methane-forming microorganisms immobilized thereon with a process liquid,
c) applying a mixture of gaseous substrates to the reactor, wherein the mixing ratio of the substrates in the gas phase is selected in such a way that the dissolution of the gaseous substrates in the process liquid leads to a stoichiometric ratio of the gaseous substrates dissolved in the process liquid,
d) discharging the gas mixture produced from the reactor.

2. Method for methanation of gaseous substrates, according to claim 1, **characterized in that** the gaseous substrates are selected from hydrogen, carbon dioxide, hydrocarbons, hydrocarbon compounds which contain optional heteroatoms selected from oxygen, nitrogen, sulfur or halogens, and from their mixtures.

3. Method for methanation of gaseous substrates, according to claim 1 or 2, **characterized in that** two or more reactors are provided in step a), wherein the reactors are connected in series with respect to the gas transport.

4. Method for methanation of gaseous substrates, according to claim 3, **characterized in that** in step b) the process liquid is conducted in countercurrent, cocurrent, crosscurrent or other flow variants with respect to the direction of transport of the gas mixture.

5. Method for methanation of gaseous substrates, according to one of claims 1 to 4, **characterized in that** in step c) the loading of the reactor with the mixture of gaseous substrates is carried out by overpressure, the pressure being 1 to 100 bar, preferably 1 to 10 bar and particularly preferably 1 to 3 bar.

6. Method for methanation of gaseous substrates, according to one of claims 1 to 5, **characterized in that** in step c) the substrate concentration in the process liquid and/or the concentration of individual components of the gas mixture produced is measured at the outlet of the reactor, and then the mixing ratio of the gaseous substrates is selected and adjusted.

7. Method for methanation of gaseous substrates, according to one of claims 1 to 6, **characterized in that** the process liquid is circulated.

8. Method for methanation of gaseous substrates, according to one of claims 1 to 7, **characterized in that** the process liquid is selected from nutrient solutions for methane-forming microorganisms, externally generated hydrolysates, drains from biogas plants and their mixtures as well as process liquids that exhibit the required trace element concentrations and/or nutrients.

9. Device for performing a method according to claim 1, comprising at least
two reactors filled with growth bodies having immobilized methane-forming microorganisms on the surface of the growth bodies,
at least one trickle device per reactor for trickling the growth bodies with a process liquid, wherein measuring devices for determining at least one component of the gaseous substrates dissolved in the process liquid being provided at the inlet and/or outlet of the trickle device, per reactor at least one inlet device for charging the reactor with a mixture of gaseous substrates, wherein the reactors optionally comprise at least one inlet for a gaseous substrate component and/or a mixing device for gases, and
per reactor at least one discharge device for discharging the gas mixture produced, wherein the discharge device optionally opens into the inlet device of an adjacent reactor.

10. Device for performing a method according to claim 1, comprising at least
a reactor with at least one essentially cylindrical area, the reactor being arranged horizontally with respect to the longitudinal axis of the cylindrical area,
at least two growth bodies, which are rotatable disks that are formed coaxially to one another and are arranged in the direction of the longitudinal axis of the cylindrical region of the reactor an inlet device for charging the reactor with a mixture of gaseous substrates, wherein the inlet device comprises at least one supply device for a gaseous substrate component and/or a mixing device for gases,
a discharge device for discharging the generated gas mixture.

## Revendications

1. Procédé de méthanisation de substrats gazeux, dans lequel les étapes suivantes sont mises en œuvre:
a) prévoir un réacteur rempli de corps de croissance ayant des microorganismes formant du méthane immobilisés sur la surface des corps de croissance;
b) ruisseller les corps de croissance ayant des micro-organismes formant du méthane immobilisés sur eux avec un liquide de traitement,
c) charger le réacteur avec un mélange de substrats gazeux, le rapport de mélange des substrats en phase gazeuse étant choisi de telle façon que la dissolution des substrats gazeux dans le liquide de traitement conduise à un rapport stoechiométrique des substrats gazeux dissous dans le liquide de traitement,
d) évacuation du mélange gazeux produit par le réacteur.

2. Procédé de méthanisation de substrats gazeux selon la revendication 1, **caractérisé en ce que** les substrats gazeux sont choisis parmi l'hydrogène, le dioxyde de carbone, les hydrocarbures, les composés hydrocarbonés contenant des hétéroatomes facultatifs choisis parmi l'oxygène, l'azote, le soufre ou les halogènes, et parmi les mélanges de ceux-ci.

3. Procédé de méthanisation de substrats gazeux selon la revendication 1 ou la revendication 2, **caractérisé en ce que** deux ou plusieurs réacteurs sont prévus à l'étape a), les réacteurs étant connectés en série en ce qui concerne le transport de gaz.

4. Procédé de méthanisation de substrats gazeux selon la revendication 3, **caractérisé en ce que**, à l'étape b), le liquide de traitement est conduit à contre-courant, à co-courant, à courant transversal ou dans d'autres variantes d'écoulement par rapport à la direction de transport du mélange gazeux.

5. Procédé de méthanisation de substrats gazeux selon l'une des revendications 1 à 4, **caractérisé en ce que** à l'étape c), le chargement du réacteur avec le mélange de substrats gazeux est réalisé avec une surpression, la pression étant de 1 à 100 bars, de préférence de 1 à 10 bars et de manière particulièrement préférée de 1 à 3 bars.

6. Procédé de méthanisation de substrats gazeux selon l'une quelconque des revendications 1 à 5, **caractérisé en ce qu'**à l'étape c), la concentration des substrats dans le liquide de traitement et/ou la concentration des différents constituants du mélange gazeux produit est mesurée à la sortie du réacteur, puis le rapport de mélange des substrats gazeux est ensuite choisi et ajusté.

7. Procédé de méthanisation de substrats gazeux selon l'une des revendications 1 à 6, **caractérisé en ce que** le liquide de traitement est mis en circulation.

8. Procédé de méthanisation de substrats gazeux selon l'une quelconque des revendications 1 à 7, **caractérisé en ce que** le liquide de traitement est choisi parmi les solutions nutritives pour les micro-organismes formant du méthane, les hydrolysats produits de manière externe, les effluents des installations de biogaz et les mélanges de ceux-ci, ainsi que parmi les liquides de traitement qui présentent les concentrations en oligo-éléments et/ou les nutriments nécessaires.

9. Appareil pour la mise en oeuvre d'un procédé selon la revendication 1, comprenant au moins
deux réacteurs remplis de corps de croissance avec des microorganismes formant du méthane immobilisés à la surface des corps de croissance,
par réacteur, au moins un dispositif de ruissellement pour le ruissellement des corps de croissance avec un liquide de traitement, des dispositifs de mesure étant prévus à l'entrée et/ou à la sortie du dispositif d'arrosage pour la détermination d'au moins un composant des substrats gazeux dissous dans le liquide de traitement,
par réacteur, au moins un dispositif d'admission pour charger le réacteur avec un mélange de substrats gazeux, ayant éventuellement au moins une alimentation pour un composant de substrat gazeux et/ou un dispositif de mélange des gaz, et
par réacteur, au moins un dispositif d'évacuation pour l'évacuation du mélange gazeux produit, le dispositif d'évacuation débouchant éventuellement dans le dispositif d'admission d'un réacteur adjacent.

10. Appareil pour la mise en œuvre d'un procédé selon la revendication 1, comprenant au moins
un réacteur ayant au moins une zone sensiblement cylindrique, le réacteur étant disposé horizontalement par rapport à l'axe longitudinal de la zone cylindrique,
au moins deux corps de croissance qui se présentent sous la forme de disques rotatifs coaxiaux l'un par rapport à l'autre et qui sont disposés dans la direction de l'axe longitudinal de la zone cylindrique du réacteur, un dispositif d'admission pour l'alimentation du réacteur avec un mélange de substrats gazeux, celui-ci comprenant éventuellement au moins une alimentation pour un composant de substrat gazeux et/ou un dispositif de mélange de gaz,
un dispositif d'évacuation pour évacuer le mélange gazeux produit.
